Europäisches Patentamt

European Patent Office

Office européen des brevets

(11) Publication number: **0 267 051 B1**

(12) **EUROPEAN PATENT SPECIFICATION**

(45) Date of publication of patent specification: **16.02.94** (51) Int. Cl.⁵: **A61K 9/70, A61L 15/16**

(21) Application number: **87309858.6**

(22) Date of filing: **06.11.87**

(54) **Transdermal administration of amines.**

(30) Priority: **07.11.86 US 928922**

(43) Date of publication of application:
**11.05.88 Bulletin 88/19**

(45) Publication of the grant of the patent:
**16.02.94 Bulletin 94/07**

(84) Designated Contracting States:
**AT BE CH DE FR GB IT LI NL SE**

(56) References cited:
EP-A- 0 131 790    EP-A- 0 262 753
WO-A-86/00806    FR-A- 2 397 190
GB-A- 2 140 019    US-A- 2 735 799
US-A- 3 304 230    US-A- 4 262 003

Description of Habitrol(TM) and Prostep(TM) transdermal systems for the systemic delivery of nicotine

(73) Proprietor: **Purepac, Inc.**
**200 Elmora Avenue**
**Elisabeth, New Jersey 07207(US)**

(72) Inventor: **Brown, Larry R.**
**1232 Beacon Street**
**Brookline, MA 02146(US)**
Inventor: **Cline, John F.**
**28 Concord Street**
**Maynard, MA 01754(US)**
Inventor: **Davidson, S. James**
**56 Beals Street**
**Brookline, MA 02146(US)**

(74) Representative: **Bizley, Richard Edward et al**
**BOULT, WADE & TENNANT**
**27 Furnival Street**
**London EC4A 1PO (GB)**

Rank Xerox (UK) Business Services
(3.10/3.09/3.3.3)

EP 0 267 051 B1

EP 0 267 051 B1

**Description**

This invention relates to a composition and article for use in transdermal or percutaneous administration to humans of a physiologically active amine and pertains more specifically to a composition and article for application to skin for systemic administration from a composition comprising a salt of said amine with a fatty acid having 8 to 20 carbon atoms, diluted with a nonpolar nonvolatile solvent.

It has long been known that various medicaments can be transported through the skin of a human to provide systemic and prolonged administration of the medicament. In the case of physiologically active amines, however, application of the pure amine to the skin frequently causes irritation, whereas application of the commonly used amine salts such as the hydrochloride, maleate or tartrate suppresses irritation but results in a relatively low rate of transport or penetration through the skin.

The transdermal adminsitration of various amines through the skin using a nonvolatile, nonpolar solvent such as, for example mineral oil as a vehicle is known and is described in EP-A-0262753, FR-A-2,397,190, GB-A-2,140,019 and US-A-2,735,799.

It has now been found that diluting physiologically active amines, whether irritating to the skin or not, or salts thereof which are soluble in nonpolar liquids, with nonpolar nonvolatile solvents enhances the rate of transport or flux of the amine through the skin with which it is placed in contact so that a much smaller area of skin contact suffices to maintain a pharmacologically effective concentration of the amine in the system. Such solvents are generally more lipophilic than hydrophilic in nature.

The problem of the skin irritability of physiologically active amines is addressed in WO-A-86/00806. However the solution provided in this document is to choose an amine such as azatadine, which does not demonstrate skin irritation and use this in preference to other antihistamines. There is however no teaching of how the irritancy of amines may be reduced for the purpose of transdermal administration.

It has now been found that salts of skin-irritating amines with fatty acids having 8 to 20 carbon atoms cause little or no irritation or inflammation of the skin and that the amines are transported through the skin at high rates from dilutions of such salts with nonpolar, nonvolatile solvents. Amines are transported only at much lower rates from dilutions of salts of the amines with more polar acids such as the maleates or tartrates. The present invention makes it possible to produce and maintain with little or no irritation a therapeutically and systemically effective concentration of a physiologically active amine in a human by transdermal administration thereof using an area of application of practical size.

Although it previously has been proposed in Abramson U.S. Patent 3,304,230 to provide solutions of salts of physiologically active amine with monocarboxylic fatty acids having from 4 to 18 carbon atoms dissolved in aerosol compositions which include various highly volatile solvents or propellants, such solutions are ineffective for the purpose of the present invention because of the virtually immediate loss of solvent after application to the skin.

In accordance with one aspect of the invention there is provided a composition for the transdermal administration of a skin irritating physiologically active amine, comprising:

a nonirritating salt of the amine, prepared by combining the amine with a stoichiometric molar excess of a fatty acid of from 8 to 20 carbon atoms, and

between 25 and 90 weight percent of a nonirritating, nonvolatile, nonpolar solvent.

Preferably the fatty acid is an unsaturated fatty acid.

The composition of the invention is adapted to be maintained in contact with human skin to produce and maintain a therapeutically and systemically effective concentration of said amine in said human. Preferably said amine is an antihistamine and the composition includes an excess of as much as 200% of said acid over the stoichiometric amount required to form said salt.

In a second aspect the invention includes articles adapted to be maintained in contact with human skin to provide systemic adminstration of a physiologically active amine in said human, said articles comprising the aforesaid composition absorbed in a solid porous water-insoluble polymeric matrix.

In accordance with a third aspect the invention provides the use of a composition in the preparation of a medicament for the transdermal administration of a skin irritating physiologically active amine to a human, wherein the composition comprises:

a nonirritating salt of the amine, prepared by combining the amine with a stoichiometric molar excess of a fatty acid of from 8 to 20 carbon atoms, and

between 25 and 90 weight percent of a nonirritating, nonpolar nonvolatile solvent.

Any physiologically active amine may be used in the present invention. Particularly preferred are the antihistamines and adrenergic amines. Among suitable amines are diphenhydramine, bromodiphenhydramine, doxylamine, phenyltoloxamine, carbinoxamine, methapheniline, pyrilamine, tripelennamine, methapyrilene, chlorothen, thenyldiamine, methafurylene, thonzylamine, pheniramine, chlorpheniramine,

2

EP 0 267 051 B1

brompheniramine, dexbrompheniramine, pyrrobutamine, promethazine, pyrathiazine, trimeprazine, methdilazine, isothipendil, ephedrine, epinephrine, norepinephrine, isoproterenol, phenylephrine, butanefrin, amphetamine, vonedrine, cyclizine, clorcyclizine, meclizine, pilocarpene, nicotine, scapolamine, chonidine, nephedapine, diphenylpyraline, phenindamine, dimethypyrindene and clemizole and the like.

Whether or not an amine is irritating can readily be determined by a conventional patch test involving maintaining the amine in contact with the skin for 24 hours, then observing the skin. If no irritation such as permanent injury, edema, pain, itching or blistering appears (although mild erythema or reddening may occur), the amine is nonirritating for the purpose of the present invention.

The solvents which can be used in all aspects of the present invention are organic nonpolar nonvolatile solvents, particularly those having dielectric constants less than 4 and boiling points above 150°C. More volatile solvents are undesirable because of their tendency to evaporate while in contact with the skin. In the absence of solvent the rate of transport of the drug through the skin is so low, in many cases, as to be impractical. The rate of transport or flux of the drug through the shin must be sufficiently high so that a pharmaceutically effective systemic concentration can be provided using a skin contact area of reasonable size. In general, the maximum area of skin contact which is practical for a single application is no more than $123cm^2$ ($20in^2$) and preferably less than $39cm^2$ ($6in^2$), although in some cases two or more applications can be employed simultaneously on separate skin areas. Since it is well known that the therapeutically effective systemic dose varies depending upon the specific bioactivity and pharmacokinetics of the particular amine being used and the weight of the patient, the minimum flux or transport rate required can readily be determined by simple arithmetic for an applicator of given area.

The rate of transport or flux of an amine through the skin can readily be determined in vitro by placing the amine or amine salt composition, with the amine suitably labeled, for example with tritium to a final specific activity of 300 disintegrations per minute per microgram, in contact with a specimen of human epidermis, e.g. human breast epidermis, with intact stratum corneum, mounted in a conventional diffusion cell providing an exposed skin surface area of $0.33 cm^2$ each. The receptor chamber of each cell is swept with a stream of 0.1 M phosphate buffered (pH 7.4) isotonic saline in direct contact with the rear surface of the epidermal sample, the flow rate being maintained at 3 mL, per hour. Samples of the saline are collected at two hour intervals and assayed by a liquid scintillation counter, applying the appropriate quench correction procedure. Steady state transport or flux of the drug through the epidermis is generally observed in less than two hours.

In addition to the foregoing characteristics of the solvent, it must itself be non-irritating to the skin and chemically inert to the amine or salt. Among suitable solvents are hydrocarbons such as mineral oil, alcohols such as benzyl alcohol, dodecyl alcohol, esters such as isopropyl myristate, isopropyl oleate, methyl stearate, tributyl citrate, ethyl laurate, diethyl maleate and similar solvents which have seven or more carbon atoms in the molecule.

The relative proportions of amine salt and solvent present may be varied over a wide range. The minimum amount of solvent needed is the amount required to dissolve the amine salt if the two are not miscible liquids. In general, it is found that the flux of the amine through the skin at first increases as the proportion of solvent increases, reaching a maximum at a relative proportion or concentration which differs depending upon the specific amine salt and specific solvent present, and then decreases. Moreover, as the relative amount of solvent increases further, a larger volume of the dilution and a larger area of contact with the skin is required to maintain the desired systemic concentration of amine for the desired time period. Within these broad limits the optimal ratio or proportion of solvent to amine salt can readily be determined by simple experimentation in the case of any particular combination of solvent with amine salt. In general, satisfactory results are obtained with compositions containing 25 to 90% by weight of solvent based on the total weight of solvent and amine salt with best results often obtained at 40 to 60% solvent by weight.

The fatty acids which are effective in the amine salts included in the present invention include octenoic, undecylenic, lauric, linoleic, oleic, myristic, stearic, isostearic, and the like. The amine salt can be employed in pure form, in which the amine and the fatty acid component are present in stoichiometric proportions. However although an excess of fatty acid tends to decrease the rate of transport of the amine through the skin, it is desirable to use an excess of fatty acid because such excess acid reduces the tendency toward skin irritation. In general, an excess above about 200% molar percent of acid offers no advantage. A 100% molar excess acid composition in the case of chlorpheniramine provides a reasonable balance between non-irritation and high transport rate of the amine through the skin.

For effective systemic administration of the amines, the dilution or solution must be maintained in contact with the skin for a prolonged period of time. This can be accomplished, for example, by thickening the solution to the point where it does not readily flow and approaches a solid in properties, either by appropriate selection of solvent or by adding appropriate thickeners or gelling agents to the solution. It can

3

also be accomplished by dispersing into the organic solution an aqueous medium to form a very viscous or gel-like water-in-oil emulsion or slurry. In either of the foregoing cases the substantially non-flowing solution can simply be applied to or coated on the skin; preferably some mechanical protection in the form of a cover of bandage is provided to prevent it from being wiped off. Another method for maintaining contact between the solution and the skin is to compound the solution with a suitable inert adhesive to produce a semisolid adhesive layer combining in one component the required payload of amine salt, the nonpolar diluent, and dermal adhesive properties. Such an adhesive layer can usefully be backed with a nonpermeable covering such as foil or plastic film to confine the drug and avoid adhesion to other surfaces. The organic solution can also be maintained in place by imbibing or absorbing it in a suitable solid carrier such as an absorbent pad of fibrous material or a porous water-insoluble polymeric matrix, the latter being preferred. It is particularly advantageous to incorporate the drug solution in a gelled cellulose triacetate matrix, as described, for example, in Nichols U.S. Patent 3,846,404.

In general, suitable carriers are porous, preferably microporous, water-insoluble materials throughout which the amine solution can be distributed or dispersed so that a supply of the amine is maintained in contact with the skin. In a preferred embodiment the carrier is provided with means for maintaining it in contact with the skin, such as a layer of adhesive. The adhesive may extend only along part or all of the periphery of the face of the carrier, or it may, if it is sufficiently permeable, cover the skin contacting face of the carrier. In the simplest case, a separate strip of adhesive tape may be employed to maintain the carrier in place. Among suitable carriers are absorbent paper, fibrous batts such as cotton batting, and various porous or microporous polymeric gel compositions such as partially cross-linked polyvinyl alcohol, polyvinyl pyrrolidone, or polyacrylamide; and porous or microporous gels of cellulose esters or ethers including cellulose acetate, cellulose butyrate, cellulose nitrate, and the like. Particularly preferred is microporous cellulose triacetate gel, as pointed out above.

Overall rate of transport of the amine through the skin, hence the systemic concentration level of the amine, can be adjusted by varying the area of the skin with which the solution is in contact, as pointed out above, or by varying the type and amount of fatty acid and/or solvent present, while the length of time during which a desired level of concentration is maintained can be adjusted by varying the amount of amine present.

The following specific examples will serve to illustrate the invention without acting as a limitation upon its scope.

Examples

Chlorpheniramine, an antihistaminic amine widely used in the form of its maleate salt, was tested for skin irritancy by absorbing the pure amine (chlorpheniramine base) in a porous cellulose triacetate film (0.20 inch 0.48 cm thick) prepared as described in U.S. Patent 3,846,404 (MA-92 POROPLASTIC film). Patches of the saturated film 2 cm$^2$ in area were placed by test subjects on their own forearm skin and held in place by clear, nonocclusive adhesive (Tegaderm), allowing constant observation of the skin under the patch. After 17 hours, severe irritation including blistering was observed in all subjects.

The pure chlorpheniramine was also converted to a salt of a fatty acid by stirring the two together in stoichiometric (equimolar) proportions, and the irritancy of the resulting salt was determined by the foregoing procedure.

Additional specimens of salts were prepared containing an excess of fatty acid above the stoichiometric amount and tested as described above.

In each case the effect on the skin was rated by visual observation on a scale from 1 (no visible change) to 7 (severe irritation with marked reddening, edema and blistering). The results were as follows, the molar ratio of amine to fatty acid for each salt being given in parentheses:

4

## TABLE I

| Formulation | | Number of Skin Tests | Irritation Rating (1 - 7) |
|---|---|---|---|
| 1. | FREE BASE | | |
| | a. Chlorpheniramine Base | 2 | 7.0 |
| | b. Chlorpheniramine Base, 23.5% solution in isopropyl oleate (IPO) | 2 | 6.5 |
| 2. | SATURATED FATTY ACID SALTS | | |
| | a. Chlorpheniramine Stearate (1:2M) | 4 | 4.0 |
| | b. Chlorpheniramine Myristate (1:2M) | 2 | 3.0 |
| | c. Chlorpheniramine Isostearate (1:2M) | 1 | 3.5 |
| 3. | UNSATURATED FATTY ACID SALTS | | |
| | a. Chlorpheniramine Oleate (1.3M) | 2 | 1.0 |
| | b. Chlorpheniramine Oleate (1.:2M) | 21 | 2.3 |
| | c. Chlorpheniramine Oleate (1:1M) | 26 | 3.5 |
| | d. Chlorpheniramine Undecylenate (1.3M) | 2 | 1.0 |
| | e. Chlorpheniramine Undecylenate (1.2M) | 2 | 2.0 |
| | f. Chlorpheniramine Undecylenate (1.:2M) in 70% IPO | 14 | 1.4 |
| | g. Chlorpheniramine Octenoate (1.2M) | 2 | 1.5 |
| | h. Chlopheniramine Linoleate (1:1M) | 1 | 3.0 |
| 4. | CARBOXYLIC ACID SALTS | | |
| | a. Chlorpheniramine Maleate (1:1M) | 9 | 1.0 |
| | b. Chlorpheniramine Salicylate (1:1M) | 2 | 1.0 |

Chlorpheniramine labelled with tritium was prepared by first synthesizing an unsaturated precursor, 1-(p-chlorophenyl-1-(2-pyridyl)-3-dimethylamino propene by the procedure of Adamson et al., J.Chem.Soc., 1957. The compound was tritiated by reaction with tritium over Raney nickel catalyst as described by Adkins et al., Organic Chemistry, Vol. I, Chapter 9 (New York, 1943). The labelled chlorpheniramine thus produced was purified by thin layer chromatography and high pressure liquid chromatography. Chlorpheniramine labelled to a final specific activity of 3 disintegrations per minute per microgram was then used to prepare salts as listed above.

The foregoing fatty acid salts all displayed a rate of transport or flux through human skin when measured as described above in a diffusion cell which was substantially less than that of the free amine (40 $\mu$g/cm$^2$/hr), ranging from about 10-20 $\mu$g/cm$^2$/hr.; the maleate and salicylate salts displayed extremely low rates, less than 6 $\mu$g/cm$^2$/hr. However, when diluted with nonpolar nonvolatile solvents in amounts ranging from 25-90% by weight based on the total weight of solvent and amine salt, the flux rate was increased, as shown by the following table:

**TABLE II**

| Chlorpheniramine | | Undecylenic Acid | | Nonpolar compound | Solvent Base:Acid | | molar ratio | Flux µg/cm²/hr |
|---|---|---|---|---|---|---|---|---|
| wt% | mole% | wt% | mole% | | wt% | mole% | | |
| 2.1 | 2.5 | 2.9 | 4.9 | Isopropyl Oleate | 95.0 | 92.6 | 1:2 | 17.4 |
| 6.5 | 7.1 | 8.9 | 14.4 | " | 84.6 | 78.5 | 1:2 | 40.7 |
| 12.7 | 11.6 | 17.1 | 23.4 | " | 70.2 | 65.0 | 1:2 | 43.5 |
| 14.0 | 14.3 | 18.8 | 28.5 | " | 67.0 | 57.2 | 1:2 | 27.4 |
| 27.3 | 24.2 | 36.9 | 48.9 | " | 35.7 | 26.9 | 1:2 | 12.9 |
| 42.3 | 33.0 | 57.7 | 67.0 | None | --- | --- | 1:2 | 11.1 |
| 31.2 | 26.6 | 42.7 | 54.5 | Isopropyl Myristate | 26.1 | 18.8 | 1:2 | 17.8 |
| 20.4 | 19.3 | 28.0 | 39.3 | " | 51.7 | 41.4 | 1:2 | 39.6 |
| 8.5 | 9.3 | 11.7 | 18.5 | " | 79.7 | 72.2 | 1:2 | 53.6 |
| 100.0 | 100.0 | --- | --- | None | --- | --- | 1:0 | 39.5 |

As can be seen from these results, the amount of solvent producing optimum flux rate varies depending upon the identity of the specific solvent. In the case of isopropyl oleate and isopropyl myristate, the optimum flux rate exceeded that of the pure or free amine as well as that of the pure amine salt.

Similar results are obtained in the case of dexchlorpheniramine, brompheniramine, and dexbrompheniramine salts.

7

Example 2

A salt of diphenhydramine was prepared by stirring it with an equimolar quantity of linoleic acid and was found to be non-irritating by the patch test. Samples of the salt were diluted with varying amounts of isopropyl myristate and the flux was measured as described in Example 1, with the following results:

TABLE III

| Diphenhydramine | | Linoleic Acid | | Isopropyl Myristate | | Base:Acid molar ratio | Flux $\mu$g/cm$^2$/hr |
|---|---|---|---|---|---|---|---|
| wt% | mole% | wt% | mole% | wt% | mole% | | |
| 12.0 | 12.7 | 13.2 | 12.7 | 74.8 | 74.6 | 1:1 | 21.0 |
| 23.7 | 25.0 | 26.0 | 25.0 | 50.3 | 50.0 | 1:1 | 61.8 |
| 35.5 | 37.3 | 39.0 | 37.3 | 25.5 | 25.4 | 1:1 | 29.2 |

The flux determined for the salt in the absence of solvent was 6.0 $\mu$g/cm$^2$/hr.

Example 3

Nicotine was found by patch test to be irritating to the skin. A salt was prepared by stirring the nicotine with the stoichiometric amount of isostearic acid (a 1:2 molar proportion) and was found to be non-irritating by patch test. Dilutions of the salt with varying amounts of isopropyl myristate solvent were also prepared, and the flux was measured as described in Example 1 with the following results:

| Nicotine | | Isostearic Acid | | IPM | | FLUX $\mu$g/cm$^2$/hr |
|---|---|---|---|---|---|---|
| wt% | mole% | wt% | mole% | wt% | mole% | |
| 22.2 | 33.3 | 77.8 | 66.7 | 0.0 | 0.0 | 40.3 |
| 16.6 | 25.6 | 58.4 | 51.3 | 25.0 | 23.1 | 100.3 |
| 11.1 | 17.5 | 38.9 | 35.1 | 50.0 | 47.4 | 98.8 |
| 5.6 | 9.0 | 19.4 | 18.0 | 75.0 | 73.0 | 57.5 |

In general and in summary, the invention provides the use of a physiologically active irritating amine, e.g., an antihistamine, in preparing a medicament for administration systemically to a human by providing a dilution of a salt of said amine with a fatty acid having 8 to 20 carbon atoms dissolved in an effective amount of a nonpolar nonvolatile solvent, and maintaining said dilution in a form adapted to contact the skin of said human.

**Claims**

1. A composition for the transdermal administration of a skin irritating physiologically active amine, comprising:
   a nonirritating salt of the amine, prepared by combining the amine with a stoichiometric molar excess of as much as 200% of a fatty acid of from 8 to 20 carbon atoms, and
   between 25 and 90 weight percent of a nonirritating, nonvolatile, nonpolar solvent.

2. The composition of claim 1 wherein the fatty acid is an unsaturated fatty acid.

3. The composition of claim 1 or claim 2 in which the solvent is selected from the group consisting of mineral oil and esters.

4. The composition of any preceding claim in which the amine is an antihistamine or nicotine.

5. An article adapted to be maintained in contact with human skin to administer a pharmacologically active amine, said article comprising:
   the composition of any one of claims 1 to 4 absorbed in a solid, porous water-insoluble carrier.

6. An article as claimed in claim 5 in which the carrier comprises a polymeric matrix.

7. An article as claimed in claim 6 in which the polymeric matrix comprises cellulose triacetate.

8. The use of a composition in the preparation of a medicament for the transdermal administration of a skin irritating physiologically active amine to a human, wherein the composition comprises:
a nonirritating salt of the amine, prepared by combining the amine with a stoichiometric molar excess of a fatty acid of from 8 to 20 carbon atoms, and
between 25 and 90 weight percent of a nonirritating, nonpolar nonvolatile solvent.

9. The use of claim 8 wherein the fatty acid is an unsaturated fatty acid.

10. The use of claim 8 or claim 9 in which the composition is absorbed into a solid, porous water insoluble carrier designed to contact the skin of the human.

11. The use of claim 10 in which the carrier comprises a polymeric matrix.

12. The use of claim 11 in which the polymeric matrix comprises cellulose triacetate.

13. The use claimed in any one of claims 8 to 12 in which the solvent is selected from the group consisting of mineral oil and esters.

**Patentansprüche**

1. Zusammensetzung zur transkutanen Verabreichung eines hautreizenden, physiologisch aktiven Amins, umfassend:
ein nicht-reizendes Salz des Amins, hergestellt durch Vereinigen des Amins mit einem stöchiometrisch molaren Überschuß von bis zu 200 % einer Fettsäure von 8 bis 20 Kohlenstoffatomen, und
zwischen 25 und 90 Gew.-% eines nicht-reizenden, nicht-flüchtigen, unpolaren Lösungsmittels.

2. Zusammensetzung nach Anspruch 1, worin die Fettsäure eine ungesättigte Fettsäure ist.

3. Zusammensetzung nach Anspruch 1 oder 2, worin das Lösungsmittel aus der Gruppe, bestehend aus Mineralöl und Estern, ausgewählt ist.

4. Zusammensetzung nach einem der vorhergehenden Ansprüche, worin das Amin ein Antihistamin oder Nikotin ist.

5. Gegenstand, der eingerichtet ist, um in Kontakt mit menschlicher Haut gehalten zu werden, zur Verabreichung eines pharmakologisch aktiven Amins, wobei der Gegenstand umfaßt:
eine Zusammensetzung nach einem der Ansprüche 1 bis 4, absorbiert in einem festen, porösen, wasserunlöslichen Träger.

6. Gegenstand nach Anspruch 5, worin der Träger eine Polymermatrix umfaßt.

7. Gegenstand nach Anspruch 6, worin die Polymermatrix Cellulosetriacetat umfaßt.

8. Verwendung einer Zusammensetzung bei der Herstellung eines Arzneimittels zur transkutanen Verabreichung eines hautreizenden, physiologisch aktiven Amins an einen Menschen, worin die Zusammensetzung umfaßt:
ein nicht-reizendes Salz des Amins, hergestellt durch Vereinigen des Amins mit einem stöchiometrisch molaren Überschuß einer Fettsäure von 8 bis 20 Kohlenstoffatomen, und
zwischen 25 und 90 Gew.-% eines nicht-reizenden, unpolaren, nicht-flüchtigen Lösungsmittels.

9. Verwendung nach Anspruch 8, worin die Fettsäure eine ungesättigte Fettsäure ist.

10. Verwendung nach Anspruch 8 oder 9, worin die Zusammensetzung in einem festen, porösen, wasserunlöslichen Träger absorbiert ist, der zum Kontaktieren der Haut des Menschen eingerichtet ist.

**11.** Verwendung nach Anspruch 10, worin der Träger eine Polymermatrix umfaßt.

**12.** Verwendung nach Anspruch 11, worin die Polymermatrix Celluosetriacetat umfaßt.

**13.** Verwendung nach einem der Ansprüche 8 bis 12, worin das Lösungsmittel aus der Gruppe, bestehend aus Mineralöl und Estern, ausgewählt ist.

**Revendications**

**1.** Composition destinée à l'administration transdermique d'une amine physiologiguement active irritante pour la peau, comprenant :

un sel non irritant de l'amine, préparé par combinaison de l'amine avec un excès stoechiométrique molaire allant jusqu'à 200 % d'un acide gras ayant 8 à 20 atomes de carbone,

et

entre 25 et 90 pour cent en poids d'un solvant non polaire, non volatil, non irritant.

**2.** Composition suivant la revendication 1, dans laquelle l'acide gras est un acide gras non saturé.

**3.** Composition suivant la revendication 1 ou la revendication 2, dans laquelle le solvant est choisi dans le groupe comprenant une huile minérale et des esters.

**4.** Composition suivant l'une quelconque des revendications précédentes, dans laquelle l'amine est une anti-histamine ou la nicotine.

**5.** Article conçu pour être maintenu au contact de la peau humaine afin d'administrer une amine pharmacologiquement active, ledit article comprenant :

la composition suivant l'une quelconque des revendications 1 à 4 absorbée dans un support solide poreux insoluble dans l'eau.

**6.** Article suivant la revendication 5, dans lequel le support comprend une matrice polymérique.

**7.** Article suivant la revendication 6, dans lequel la matrice polymérique comprend du triacétate de cellulose.

**8.** Utilisation d'une composition dans la préparation d'un médicament destiné à l'administration transdermique à un être humain d'une amine physiologiquement active irritante pour la peau, dans laquelle la composition comprend :

un sel non irritant de l'amine, préparé par combinaison de l'amine avec un excès molaire stoechiométrique d'un acide gras de 8 à 20 atomes de carbone

et

entre 25 et 90 pour cent en poids d'un solvant non volatil, non polaire, non irritant.

**9.** Utilisation suivant la revendication 8, dans laquelle l'acide gras est un acide gras non saturé.

**10.** Utilisation suivant la revendication 8 ou la revendication 9, dans laquelle la composition est absorbée dans un support solide poreux insoluble dans l'eau conçu pour rester au contact de la peau d'un être humain.

**11.** Utilisation suivant la revendication 10, dans laquelle le support comprend une matrice polymérique.

**12.** Utilisation suivant la revendication 11, dans laquelle la matrice polymérique comprend du triacétate de cellulose.

**13.** Utilisation suivant l'une quelconque des revendications 8 à 12, dans laquelle le solvant est choisi dans le groupe comprenant une huile minérale et des esters.